(19) **Europäisches Patentamt**

**European Patent Office**

**Office européen des brevets**

(11) **EP 1 666 542 A1**

(12) **EUROPEAN PATENT APPLICATION**
published in accordance with Art. 158(3) EPC

(43) Date of publication:
**07.06.2006 Bulletin 2006/23**

(51) Int Cl.:
*C09C 1/32* (1968.09)  *C09C 3/06* (1974.07)
*C09D 11/00* (1968.09)  *C09D 201/00* (1990.01)
*C08L 101/00* (1985.01)  *C08K 9/02* (1990.01)

(21) Application number: **04787952.3**

(22) Date of filing: **21.09.2004**

(86) International application number:
**PCT/JP2004/013769**

(87) International publication number:
**WO 2005/028567 (31.03.2005 Gazette 2005/13)**

(84) Designated Contracting States:
**DE FR GB IT**

(30) Priority: **22.09.2003 JP 2003329237**

(71) Applicant: **Nippon Sheet Glass Co.,Ltd.**
**Tokyo 105-8552 (JP)**

(72) Inventors:
• **HIRASHIMA, Takashi**
**c/o NIPPON SHEET GLASS COMPANY,**
**Minato-ku, Tokyo 1058552 (JP)**

• **FURUICHI, Toshitaka**
**c/o NIPPON SHEET GLASS COMPANY**
**Minato-ku, Tokyo 1058552 (JP)**
• **YOKOI, Koji**
**c/o NIPPON SHEET GLASS COMPANY,**
**Minato-ku, Tokyo 1058552 (JP)**

(74) Representative: **Teipel, Stephan et al**
**Lederer & Keller**
**Patentanwälte**
**Prinzregentenstrasse 16**
**80538 München (DE)**

(54) **BLUE-COLORED FLAKE, AND COSMETIC, COATING COMPOSITION, RESIN COMPOSITION AND INK COMPOSITION COMPRISING THE SAME**

(57)    The present invention provides blue colored flakes which are free from agglomeration of the ultramarine particles, and when incorporated in a cosmetic, a coating material, or the like, they are free from rough feeling, and give good use feeling such as good spread on the skin as well as good spread on a base plate, and exhibit beautiful color tone.

The blue colored flakes comprise, as a mother material, a transparent metal oxide such as silicon dioxide and aluminum oxide and, incorporated therein in a dispersed state, 5 to 60% by mass of ultramarine particles having a particle diameter of 30 to 3000 nm. The blue colored flakes have an average particle diameter of 5 to 500 μm, an average thickness of 0.1 to 5 μm, and an average aspect ratio of 5 to 300.

EP 1 666 542 A1

## Description

### Field of the Invention

The present invention relates to blue colored flakes to be added as a filler to cosmetics, coating materials, resins, films or inks. Further, the invention relates to a cosmetic, a coating material, a resin composition, and an ink composition, each containing the blue colored flakes.

### Background of the Invention

Since ultramarine has particular clear color tone which is not exhibited in other pigments, and has excellent alkali resistance and light fastness as well as excellent heat resistance and safety, it has been used as a filler for cosmetics, coating materials, resins, films or inks. However, ultramarine is apt to be easily agglomerated in the case of a granular powder form, and thus, if ultramarine is incorporated in a cosmetic, a coating material, or a resin, rough feeling (feeling like something rough) is caused, and thus there were problems that the touch feeling and smoothness for use became worsened. Further, if the granular particles of the ultramarine are agglomerated, the function of diffusing transmitted light and reflected light is decreased, resulting in failure to obtain the clear color tone particular for the ultramarine, which was a problem. Moreover, the ultramarine comprises silicic acid, alumina, sodium, and sulfur, and owing to the effect of sulfur, sulfur smell is sometimes evolved in the case where the ultramarine is rubbed, though it is slight. Therefore, its use becomes a problem with respect to cosmetics for which smell is very important and it is needed to adjust the addition amount and make the smell unnoticeable.

In order to solve the above-mentioned problem of rough feeling because of the fine particle agglomeration and improve the touch feeling and smoothness for use, those comprising spherical inorganic powder to whose surface fine particles are attached have been developed (Patent Document No. 1: Japanese Patent Application Laid-Open No. 8-217637, and Patent Document No. 2: Japanese Patent Application Laid-Open No.11-001411). They are prevented from agglomeration of fine particles by sticking fine particles of zinc oxide, etc. to the surface of the inorganic powder, however, since the inorganic powder is spherical, the inorganic powder itself is easy to agglomerate and in the case where it is added to a cosmetic, there occur problems of inferior touch feeling and smoothness for use. Further, there is another problem that the fine particles adhering to the surface of the inorganic powder are easily peeled off by mechanical friction.

Non-Patent Document No. 1 (Edited by Seishiro, ITO, "Dictionary of Pigments", p. 225 (2000), Asakura Shoten Ltd.) describes ultramarine particles coated with a silica coating. However, those ultramarine particles are useful for improvement of durability and prevention of sulfur smell , but it is impossible to prevent agglomeration and in the case where the ultramarine particles are used for cosmetics, the rough feeling is caused.

Patent Document No. 3: Japanese Patent Application Laid-Open No. 10-087433 discloses a filler comprising a platy powder of silica to which fine particles are attached. In the case of the platy powder, it hardly agglomerate itself and therefore, it is expected that a cosmetic using the filler is free from rough feeling and gives smooth use feeling.

However, only attachment of fine particles to the platy powder cannot suppress the smell in the case of using fine particles emitting smell like ultramarine.

### Disclosure of the Invention

### Problems to be Solved by the Invention

The present invention has been made in view of the above-mentioned technical problems. It is an object of the present invention to prevent agglomeration of ultramarine particles and provide blue colored flakes which can prevent rough touch feeling and give good use feeling such as good spread on the skin as well as good spread on the base plate, and exhibit beautiful color tone. Further, another object of the present invention is to provide blue colored flakes which are free from emitting sulfur smell in the case where they are incorporated in a cosmetic or the like.

### Means for Solving the Problems

Blue colored flakes of the present invention comprise a transparent metal oxide as a mother material and 5 to 60% by mass of ultramarine particles having a particle diameter of 30 to 3000 nm dispersed in the mother material.

The blue colored flakes of the present invention comprise a transparent metal oxide as a mother material and ultramarine particles incorporated in the mother material in a dispersed state. As the transparent metal oxide, silicon dioxide and aluminum oxide (dialuminum trioxide) are preferable to be used. The blue colored flakes are suitable for use as a filler for cosmetics or resin compositions, and since the outer shape is scaly, the flakes have a characteristic that agglomeration hardly occurs. Therefore, in the case where said blue colored flakes are added to a cosmetic, deterioration in use feeling such as rough feeling is prevented. The blue colored flakes have high heat resistance and acid resistance, and are used as a blue pigment having any arbitrary color tone.

The blue colored flakes of the present invention comprise ultramarine particles having a particle diameter of 30 to 3000 nm, preferably 50 to 2000 nm incorporated therein in a dispersed state. Since the blue colored flakes of the present invention which are incorporated in cosmetics, coating materials, resin compositions for films, ink compositions, or the like so as to finally become a thin film-like form are arranged in the inside of the thin film without agglomeration while being oriented in parallel to the film face of the thin film, the ultramarine particles in the inside of the flakes are arranged evenly, resulting in giving a higher effect as a pigment.

If a cosmetic, a coating material, a resin composition for a film, an ink composition, or the like contains the ultramarine particles which are not enclosed in the flakes, the ultramarine particles are agglomerated to form secondary particles having a large particle diameter. This agglomeration (secondary particle formation) of the ultramarine particles causes unevenness in the coating film. Also, since a cosmetic containing the ultramarine particles which are not enclosed in the insides of the flakes tends to cause agglomeration, the cosmetic is poor in the spreading property and its use feeling is worsened. A coating material containing the ultramarine particles which are not enclosed in the insides of the flakes is less extensive at the time of incorporation.

The blue colored flakes of the present invention have preferably an average particle diameter of 5 to 500 $\mu$m, an average thickness of 0.1 to 5 $\mu$m, and an average aspect ratio of 5 to 300; more preferably an average particle diameter of 8 to 300 $\mu$m, an average thickness of 0.2 to 2.5 $\mu$m, and an average aspect ratio of 8 to 200; and furthermore preferably an average particle diameter of 8 to 50 $\mu$m, an average thickness of 0.5 to 2.0 $\mu$m, and an average aspect ratio of 8 to 50. If the average particle diameter is less than 5 $\mu$m, the blue colored flakes tend to easily agglomerate and therefore cause unevenness. On the other hand, if the average particle diameter exceeds 500 $\mu$m, the blue colored flakes are easily broken at the time of addition as a filler, and in the case of addition to a cosmetic, the flakes deteriorate the use feeling of the cosmetic, resulting in, for example, causing rough feeling. If the average thickness is thinner than 0.1 $\mu$m, production becomes difficult and it results in problems such that the flakes are easily broken. On the other hand, if the average thickness exceeds 5 $\mu$m, in the case of addition to a coating material, the surface of a coating film becomes uneven to worsen the appearance, or in the case of addition to a cosmetic, rough feeling is caused and thus the use feeling is worsened. If the average aspect ratio is less than 5, characteristics of spherical particles tend to appear and the flakes tend to easily agglomerate. On the other hand, if the average aspect ratio exceeds 300, the flakes are crushed at the time of addition as a filler and thus the particle diameter becomes uneven and it easily results in color shading.

The average particle diameter of the blue colored flakes can be measured by a measurement apparatus for particle size distribution through laser diffraction/scattering, for example, Microtrac II (manufactured by Nikkiso Co., Ltd.) ; the average thickness by calculating a simple average of measurement results of 50 pieces of blue colored flakes can be measured by an electron microscope; and the average aspect ratio can be measured by dividing the value of the above-mentioned average particle diameter by the value of the above-mentioned average thickness.

Production of the blue colored flakes is not particularly limited, and a method of drawing a melted glass mixed with ultramarine particles into a film-like shape and the so-called sol-gel method may be employed. Among the methods, the sol-gel method is particularly suitable. For example, a method described in Japanese Patent Application Laid-Open No. 1-9803, that is, a method which involves producing a starting material liquid by dispersing ultramarine particles having an average particle diameter of 30 to 3000 nm into a metal compound solution containing a metal alkoxide or a metal organic acid salt or into a metal oxide sol (a starting material of a mother material); forming a coating film by applying the starting material liquid to the surface of a base material having a smooth face; making the film flaky by treatment, for example, drying or reaction (oxidation decomposition); and separating the flakes from the base material. In this case, a commercially available colloidal solution of a metal oxide (hereinafter, also referred to as sol solution) may be used as the metal oxide sol of the mother material. The separated flakes are fired at 200 to 800°C, and if necessary, crushed and classified to obtain blue colored flakes having an arbitrary average particle diameter. In the present invention, conventionally known additives may be added to the above starting material liquid, and the types of the additives may include disaccharides such as trehalose or the like. The amount of these additives is not particularly limited unless the purpose of the invention is inhibited, however, it is generally 3 to 60% by mass, preferably 5 to 20% by mass, to the mass of the metal oxide and ultramarine.

The shape of the base material for coating is not particularly limited, and a roll type, a belt type, and a sheet type can be exemplified. Among them, in view of the productivity of the flaky substance, the roll type or belt type is a preferable one suitable for continuous production. Examples of the material for the roll type base material are a metal such as iron and a stainless steel; a plated material such as a metal plated with chromium; a ceramic or glass containing aluminum oxide or zirconium oxide; and these materials coated with a polymer such as silicone rubber. As a material for the belt type or sheet type, there are exemplified a metal such as iron and a stainless steel; a ceramic or glass containing aluminum oxide or zirconium oxide; and a resin composition such as polyethylene terephthalate, polyimide and polyamide.

A method for applying the starting material liquid to the base material to be coated is not particularly limited, and conventionally known methods may be applied as they are. For example, a dipping method, a bar coater method, a roll coater method, a curtain coater method, and a spray method can be exemplified.

After the starting material liquid is applied to the base material to be coated, the base material or the coating film is heated to be dried, and the film is made into gel and further into a thin film. The heating method includes, for example, a method of directly heating the coating film by hot air, an infrared heater, or high frequency heating, and a method of indirectly heating the coating film by bringing the base material into contact with a heat generator. At the time of forming a thin film, since the thin film is shrunk by removal of a solvent and heating, numberless cracks are formed in the thin film. Owing to the formation of the cracks, the thin film becomes flaky. The step of separating the flakes from the base material can easily be carried out by using conventionally known proper means , for example, sliding the base material alone while pressing the flakes with a nonwoven fabric such as felt or the like, thereby to separate the base material and the flakes.

The separated flaky metal compound or metal oxide is fired, and if necessary, crushed and classified to obtain flakes having an optional average particle diameter. Although it depends on the color tone, the firing temperature and duration are preferable under such a condition that the ultramarine color is not discolored. It is preferable to keep the firing temperature at not higher than 600°C in oxygen atmosphere, and at not higher than 1200°C in non-oxidative atmosphere. In the case where the firing temperature is relatively low, for example 400°C, porous flakes are obtained, and in the case where the temperature is relatively high, for example higher than 800°C, flakes which are not porous but dense are obtained. The porous flakes have relatively low strength due to their porosity, however, the refractive index (air = 1) of the voids is much smaller than that of the mother material, and owing to the interfaces of the voids, it is expected that visible light is more scattered. Therefore, the porous flakes are preferably usable for uses requiring low stress load and high scattering intensity. The dense flakes are preferably usable for uses requiring relatively high strength and for uses in which absorption of surrounding substances in the fine pores becomes a problem.

With respect to the blue colored flakes of the present invention obtained in the above-mentioned manner, since ultramarine particles are dispersed and enclosed in the insides, even in the case of adding the flakes as a filler for various uses, the ultramarine particles are not separated from the flakes.

The content of the ultramarine particles in the blue colored flakes is 5 to 50% by mass, preferably 8 to 30% by mass. If the content is less than 5% by mass, pale blue tone but not clear blue is exhibited and the intrinsic color of ultramarine is thus deteriorated. On the other hand, if it exceeds 50% by mass, the flakes become fragile and their mechanical strength is lowered. The shape of the ultramarine particles is not particularly limited, and may be amorphous, spherical, column-like, and oval. The above-mentioned content (% by mass) of the ultramarine particles can be defined by the following equation:

$$\text{Content of ultramarine particles (\% by mass)} = \frac{\text{Mass of ultramarine particles}}{\text{Mass of blue colored flakes}} \times 100$$

Addition of the blue colored flakes of the present invention to a cosmetic gives a cosmetic having an excellent touch feeling to the skin and particular beautiful blue which the ultramarine has. Further, the sulfur smell which ultramarine has can be suppressed by processing ultramarine into flakes. The content of the blue colored flakes in the cosmetic is properly 1 to 70% by mass. If it is less than 1% by mass, the coloration effect as a blue powder is slight and on the other hand, if it exceeds 70% by mass, the coloration effect as a blue powder becomes significant to lose transparency and result in unnatural finishing. It is more preferably 3 to 50% by mass.

The blue colored flakes of the present invention may be subjected properly to hydrophobic treatment in accordance with the purpose of the cosmetic. The method for hydrophobic treatment may be treatment with a silicone compound such as a methylhydrodiene polysiloxane, a highly viscid silicone oil, or a silicon resin; treatment with a surfactant such as an anionic surfactant or a cationic surfactant; treatment with a polymer compound such as nylon, poly(methyl methacrylate), polyethylene, a fluoro resin, or a polyamino acid; treatment with a perfluoro group-containing compound, lecithin, collagen, a metal soap, an oleophilic wax, or a partial or complete ester of a polyhydric alcohol; or treatment in combination thereof. However, the treatment is not limited to these exemplified treatments, and any method capable of applying a powder to the hydrophobic treatment can be used.

The cosmetic may appropriately contain other components to be used commonly for a cosmetic beside the blue colored flakes of the present invention. Other components may include an inorganic powder, an organic powder, a pigment, a coloring agent, an oil component, an organic solvent, a resin, and a plasticizer. Examples of the inorganic powder may be talc, kaolin, sericite, muscovite, phlogopite, lepidolite, biotite, lithia mica, vermiculite, magnesium carbonate, calcium carbonate, diatomaceous earth, magnesium silicate, calcium silicate, aluminum silicate, barium silicate, barium sulfate, strontium silicate, metal tungstate, silica, hydroxyapatite, zeolite, boron nitride, and a ceramic powder.

The organic powder may include a nylon powder, a polyethylene powder, a polystyrene powder, a benzoguanamine powder, a poly(tetrafluoroethylene) powder, a distyrene-benzene polymer powder, an epoxy powder, and an acrylic powder.

The pigment may include microcrystalline cellulose, inorganic white pigments such as titanium dioxide and zinc oxide; an inorganic red type pigment such as iron oxide (red iron oxide) and iron titanate; inorganic brown type pigments such as γ-iron oxide; inorganic yellow type pigments such as yellow iron oxide and yellow earth; inorganic black type pigments such as black iron oxide and carbon black; inorganic violet type pigments such as mango violet and cobalt violet; inorganic green type pigments such as chromium oxide, chromium hydroxide, and cobalt titanate; inorganic blue type pigments such as prussian blue; pearl pigments such as titanium oxide-coated mica, titanium oxide-coated bismuth oxychloride, bismuth oxychloride, titanium oxide-coated talc, scaly foil, and colored titanium oxide-coated mica; and metal powder pigments such as aluminum powder and copper powder.

The coloring agent may include organic pigments such as Red No. 201, Red No. 202, Red No. 204, Red No. 205, Red No. 220, Red No. 226, Red No. 228, Red No. 405, Orange No. 203, Orange No. 204, Yellow No. 205, YellowNo. 401, andBlueNo. 404; organic pigments of zirconium, barium, or aluminum lakes of Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3 , or Blue No. 1: and natural coloring agents such as chlorophyll and β-carotene.

Further, the oil component may include various kinds of hydrocarbons, silicone oils, higher fatty acids, esters of fats and oils, higher alcohols, and waxes, such as squalane, liquid paraffin, vaseline, microcrystalline wax, ozokerite, ceresin, myristic acid, palmitic acid, stearic acid, oleic acid, isostearic acid, cetyl alcohol, hexadecyl alcohol, oleyl alcohol, cetyl 2-ethylhexanoate,2-ethylhexyl palmitate,2-octyldodecyl myristate, neopentyl glycol di-2-ethylhexanoate, glycerol tri-2-ethylhexanoate, 2-octyldodecyl oleate, isopropyl myristate, glycerol triisostearate, coconut fatty acid triglyceride, olive oil, avocado oil, beeswax, myristyl myristate, mink oil and lanoline.

Further, other components to be added to cosmetics may include an organic solvent such as acetone, toluene, butyl acetate and acetic acid esters; a resin such as alkyd resin and urea resin; a plasticizer such as camphor and acetyl tributyl citrate; an ultraviolet absorbent, an antioxidant, a preservative, a surfactant, a moisture agent , a fragrance , water, alcohol, and a thickener.

The cosmetic may take a variety of forms including powder, cake-like form, pencil-like form, stick form, ointment, liquid, emulsion, and cream. They may include, for example, facial cosmetics such as a lotion, an emulsion, and a cream; and make-up cosmetics such as a foundation, a lipstick, an eye shadow, a cheek rouge, an eyeliner, a nail enamel, and a mascara.

Further, like a conventional filler, this blue colored flakes may be used as a filler for coating materials or as an additive to a resin to be kneaded, or also as a filler for films or inks. In the case where the blue colored flakes are used as a coating material, it can be extended well on a coating base material and makes the underlayer invisible, and shows transparent blue. In the case of use for resin molded products, resin films, and inks, the blue colored flakes are provided with highvisible light scattering effect orbeautiful and transparent blue color. The content of the blue colored flakes in the coating material composition, the resin composition, and the ink composition is preferably 1 to 70% by mass. If it is less than 1% by mass, the visible light scattering effect is lowered, and it is difficult to obtain an underlayer hiding effect and transparent blue. If the content of such flakes in the composition exceeds 70% by mass, the gloss becomes too intense. The content of the blue colored flakes is more preferably 3 to 50% by mass.

Best Mode For Carrying Out the Invention

Hereinafter, the invention will be described more in detail with reference to Examples, however, it is not intended that the present invention be limited to the illustrated Examples.

**Examples**

The color tone of the flakes and use feeling on the skin were evaluated by the following means for blue colored flakes produced in respective Examples and Comparative Examples.

(Examples 1 to 5)

While a sol solution of silicon dioxide (silica) (Silicadol 30A; particle diameter: 20 nm; dispersant: water; manufactured by Nippon Chemical Industrial Co., Ltd.) 141 g (silica 42 g) was stirred, ultramarine particles (CB-80; particle diameter: 200 nm; manufactured by Daiichi Kasei Kogyo Co., Ltd.) 2.2 g for Example 1, 4.7 g for Example 2, 18 g for Example 3, 42 g for Example 4, and 63 g for Example 5 were added; pure water 178 g was further added; each mixture was mixed; and the particles were dispersed for 10 minutes by using a dispersion homogenizer which is previously known, thereby to produce ultramarine particles-containing silica sols. At that time, the ultramarine particles-containing silica sol was confirmed to be an alkali of pH 8 to 10. Trehalose (molecular weight 342), which is a water-soluble substance, was added at a ratio of 10% by mass to the total solid matter mass (the mass of metal oxide + mass of ultramarine) and dispersed for further 30 minutes by using a conventionally known dispersion homogenizer. The resulting sol solutions were allowed to stand at 25°C for 18 hours, and each sol solution was applied to a polyethylene terephthalate (PET) film with a width of 10 cm by a bar coater, so that the thickness of the film becomes to be 1.0 $\mu$m after drying, and then the film was put in a drying furnace at 120°C for 3 minutes to dry the sol solution. The film was taken out of the drying furnace and cooled to room temperature. At that time, the thin film was evenly stuck to the surface of the PET film. While room temperature water was sprayed to the film, felt was pressed against the thin film formed face by pinching them with a commercialized binder clip and the film alone was pulled and slid. Accordingly, the thin film was separated from the film surface. The separated flakes were recovered and dried at 120°C for 1 hour and successively fired at 400°C for 5 hours. Flakes of porous silica-ultramarine mainly containing silica were obtained, wherein the ultramarine particles were dispersed and enclosed therein. The flakes were classified by using a conventionally known apparatus to adjust the average particle diameter to 10 $\mu$m, the average thickness to 1.0 $\mu$m, and the average aspect ratio to 10.

With respect to these flakes, the touch feeling to the skin, sulfur smell, and chroma on the coated plate were measured and evaluated. The average particle diameter ($\mu$m), the average thickness ($\mu$m), and the average aspect ratio of the flakes, the content (% by mass) of the ultramarine particles dispersed and contained in the flakes, silica content (% by mass), color tone of the powder, and results of the skin touch evaluation, sulfur smell evaluation, and the chroma evaluation by sensory test are shown in Table 1. With respect to the flakes of respective Examples, in the case the content of the ultramarine particles was 5 to 60% by mass, it is found that the skin touch evaluation and sulfur smell evaluation are more excellent than those in the case the ultramarine particles themselves. In terms of the comprehensive evaluation of the skin touch evaluation, sulfur smell evaluation, and the chroma evaluation, it is understood that the content of the ultramarine particles is more preferably 10 to 50% by mass. For comparison, the ultramarine particles themselves were evaluated as Comparative Example 1 in the same manner as described above.

Table 1

|  | Example | | | | | Comparative Example 1 |
|---|---|---|---|---|---|---|
|  | 1 | 2 | 3 | 4 | 5 |  |
| Average particle diameter ($\mu$m) | 10 | 10 | 10 | 10 | 10 | - |
| Average thickness ($\mu$m) | 1 | 1 | 1 | 1 | 1 | - |
| Average aspect ratio | 10 | 10 | 10 | 10 | 10 | - |
| Ultramarine particle content (% by mass) | 5 | 10 | 30 | 50 | 60 | 100 |
| Silica content (% by mass) | 95 | 90 | 70 | 50 | 40 | 0 |
| Color tone of the powder | Pale blue | Blue | Blue | Blue | Blue | Blue |
| Skin touch | ◎ | ◎ | ◎ | ◎ | O | X |
| Sulfur smell | ◎ | ○ | ○ | ○ | Δ | X |
| Chroma | 55 | 63 | 63 | 63 | 63 | 61 |

The evaluation tests for the skin touch and sulfur smell were carried out by evaluating the skin touch and the sulfur smell according to the following standard.
Skin touch:

◎ : smoothly spread
○ : no rough feeling
Δ : slightly rough feeling
× : rough feeling

Sulfur smell:

◎ : scarcely smelt
O : slightly smelt
Δ : smelt
× : offensively smelt

With respect to chroma, the evaluation test was carried out as follows.
The blue colored flakes of Example 3 were added in a ratio of 10% by mass to an acrylic resin coating material (Acryl Auto Clear Super; solid matter about 50% by mass; manufactured by Nippon Paint Co. , Ltd.), stirred well and mixed, and the mixture was applied to shielding ratio measurement paper by an applicator with 9 mil (9/1000 inch) intervals and dried to produce a coated sheet. With respect to the chroma of the ultramarine particles of flakes of Examples 1, 2, 4, and 5 and Comparative Example 1, each coated sheet wastproduced in the same manner as described above, except that the content of the ultramarine particles in the acrylic resin became to be the same as described above. The a and b values of each coated plate were measured by a colorimeter (CR-300, manufactured by Minolta) and the chroma was calculated from the following equation.

$$\text{Chroma} = (a^2 + b^2)^{1/2}$$

Next, cosmetics were produced using the blue colored flakes produced in the respective Examples and ultramarine particles of Comparative Example 1, and sensory tests for the use feeling were carried out. The items for the sensory tests were three items; finishing impression, touch feeling at the time of application to the skin and eyelash, and beauty of blue; and the respective items were evaluated according to 5 grades from 1 to 5. The evaluation standards of the respective items are shown in Tables 2 to 4.

Table 2

| Evaluation | Finishing impression |
| --- | --- |
| 1 | very unnatural |
| 2 | unnatural |
| 3 | ordinary |
| 4 | tolerably natural |
| 5 | natural |

Table 3

| Evaluation | Touch feeling |
| --- | --- |
| 1 | rough feeling |
| 2 | slightly rough feeling |
| 3 | ordinary |
| 4 | slightly smooth |
| 5 | smooth |

Table 4

| Evaluation | Beautifulness of blue |
| --- | --- |
| 1 | uneven and no bright impression |
| 2 | no unevenness but no bright impression |
| 3 | ordinary |
| 4 | beautiful blue with slightly bright impression |
| 5 | transparent and clear blue with bright impression |

Ten panelists were employed in the following sensory tests on cosmetics. Three items of finishing impression, touch feeling, and blue beauty on the cosmetics were evaluated based on the average value of these ten panelists. Additionally, to make the evaluation results easy to be seen, the following symbols shown in Table 5 are marked according to the average values of the evaluation.

Table 5

◎ :  4.5 or higher, but up to 5.0
○ :  3.5 or higher, but less than 4.5
● :  2.5 or higher, but less than 3.5
Δ :  1.5 or higher, but less than 2.5
✕ :  1.0 or higher, but less than 1.5

(Example 6: Emulsion type mascara)

An emulsion type mascara was produced using the following respective components shown in the following Table 6.

Table 6

| (1) | hydroxyethyl cellulose | 1.0 |
|---|---|---|
| (2) | methyl p-oxybenzoate | 0.2 |
| (3) | glycerin | 0.3 |
| (4) | polyethylene glycol with high polymerization degree (average molecular weight 2,000,000) | 0.5 |
| (5) | purified water | 65.0 |
| (6) | blue colored flakes of Example 1 | 3.0 |
| (7) | triethanolamine | 3.0 |
| (8) | stearic acid | 5.0 |
| (9) | beeswax | 9.0 |
| (10) | carnauba wax | 3.0 |
| (11) | paraffin wax | 10.0 (% by mass) |

The components (1) to (5) were mixed together and evenly dissolved by heating at 75°C. The blue colored flakes of the component (6) were added to the mixture and evenly dispersed through a colloid mill. Further, the component (7) was mixed, dissolved, and heated at 75°C to make the mixture evenly heated and dissolved. The components (8) to (11) were added thereto, and the resultant mixture was emulsified and cooled to obtain an emulsion type mascara.

(Comparative Example 2)

An emulsion type mascara was produced in the same manner as in Example 6, except that the ultramarine particles of Comparative Example 1 were used in place of the blue colored flakes of the component (6) in Example 6.
The results of the sensory tests for Example 6 and Comparative Example 2 are collectively shown in Table 7.

Table 7

| | Finishing impression | Touch feeling | Beautifulness of blue color |
|---|---|---|---|
| Example 6 | ○ | ◎ | ◎ |
| Comparative Example 2 | ○ | Δ | ○ |

It is understood from Table 7 that the emulsion type mascara of the present invention is excellent in the touch feeling and the beautifulness of blue color.

(Example 7: Eye shadow)

An eye shadow was produced from the following components shown in Table 8.

Table 8

| (1) | talc | 21 |
|---|---|---|
| (2) | muscovite | 20 |
| (3) | blue colored flakes of Example 1 | 40 |
| (4) | pigment | 12 |
| (5) | squalane | 4 |
| (6) | cetyl 2-ethylhexanoate | 1.9 |

EP 1 666 542 A1

Table continued

|     |                       |                  |
| --- | --------------------- | ---------------- |
| (7) | sorbitan sesquioleate | 0.8              |
| (8) | preservative          | 0.1              |
| (9) | fragrance             | 0.2 (% by mass)  |

The above-mentioned components (1) to (4) were mixed by a Henschel mixer, and the components (5) to (9) which were heated and mixed were blown for mixing and then the mixture was milled. The resulting mixture was discharged to a medium-sized dish to obtain an eye shadow.

(Comparative Example 3)

An eye shadow was produced in the same manner as in Example 7, except that the ultramarine particles of Comparative Example 1 were used in place of the blue colored flakes of the component (3).

The results of the sensory test of Example 7 and Comparative Example 3 are collectively shown in Table 9.

Table 9

|                       | Finishing impression | Touch | Beautifulness of blue color |
| --------------------- | -------------------- | ----- | --------------------------- |
| Example 7             | O                    | ◎     | ◎                           |
| Comparative Example 3 | O                    | Δ     | O                           |

It is understood from Table 9 that the eye shadow of the present invention is excellent in the touch feeling and the beautifulness of blue color.

(Example 8: Eyeliner)

An eyeliner was produced from the following respective components shown in Table 10.

Table 10

|     |                               |                  |
| --- | ----------------------------- | ---------------- |
| (1) | non-aqueous polymer dispersion | 25.0            |
| (2) | paraffin wax                  | 2.0              |
| (3) | bentonite                     | 3.0              |
| (4) | blue colored flakes of Example | 1 2.0           |
| (5) | mica                          | 30.0             |
| (6) | Isopar                        | 38.0             |
| (7) | fragrance                     | q.s.(% by mass)  |

The above-mentioned components (1) to (7) were heated to 85°C, stirred and mixed, and then cooled to room temperature, and packed in an air-tight and brush-equipped container to produce an eyeliner.

(Comparative Example 4)

An eyeliner was produced in the same manner as Example 8, except that the ultramarine particles of Comparative Example 1 were used in place of the blue colored flakes of the component (4).

The results of the sensory test of Example 8 and Comparative Example 4 are collectively shown in Table 11.

Table 11

|                       | Finishing impression | Touch feeling | Beautifulness of blue |
| --------------------- | -------------------- | ------------- | --------------------- |
| Example 8             | ○                    | ◎             | ◎                     |
| Comparative Example 4 | ○                    | ○             | ○                     |

It is understood from Table 11 that the eyeliner of the present invention is excellent in the touch feeling and the beautifulness of blue color.

(Example 9: Nail color)

A nail color was produced from the following components shown in Table 12.

Table 12

| (1) | nitrocellulose | 18.0 |
|---|---|---|
| (2) | toluenesulfonamide resin | 6.0 |
| (3) | acetyl tributyl citrate | 6.0 |
| (4) | alkyl acrylate copolymer | 2.0 |
| (5) | isopropanol | 5.0 |
| (6) | benzyldimethylammonium hectorite | 2.0 |
| (7) | ethyl acetate | 20.0 |
| (8) | butyl acetate | q.s. |
| (9) | prussian blue | 0.1 |
| (10) | blue colored flakes of Example 1 | 10.0 (% by mass) |

After the components (1) to (4) and components (9) and (10) were kneaded by a roller mill, the components (5) to (8) were added thereto, melted, diffused, and evenly dispersed, and then the resulting mixture was packed in a prescribed container to obtain a nail color.

(Comparative Example 5)

A nail color was produced in the same manner as Example 9, except that the ultramarine particles of Comparative Example 1 were used in place of the blue colored flakes of the component (10).
The results of the sensory test of Example 9 and Comparative Example 5 are collectively shown in Table 13.

Table 13

| | Finishing impression | Touch feeling | Beautifulness of blue color |
|---|---|---|---|
| Example 9 | O | ◎ | ◎ |
| Comparative Example 5 | O | O | O |

It is understood from Table 13 that the nail color of the present invention is excellent in the touch feeling and the beautifulness of blue color.

(Example 10: Oil-based stick foundation)

An oil-based stick foundation was produced from the following components shown in Table 14.

Table 14

| (1) | blue colored flakes of Example 1 | 13.0 |
|---|---|---|
| (2) | titania | 7.0 |
| (3) | kaolin | 20.0 |
| (4) | talc | 2.0 |
| (5) | mica | 26.0 |
| (6) | red iron oxide | 1.0 |
| (7) | yellow iron oxide | 3.0 |
| (8) | blue colored flakes of Example 1 | 0.5 |
| (9) | solid paraffin | 3.0 |
| (10) | microcrystalline wax | 7.0 |
| (11) | vaseline | 15.0 |
| (12) | dimethylpolysiloxane | 3.0 |
| (13) | squalane | 5.0 |
| (14) | isopropyl palmitate | 17.0 |

Table continued

| (15) | antioxidant | q.s. |
| (16) | fragrance | q.s. (% by mass) |

The above-mentioned components (9) to (15) were dissolved at 85°C, and the components (1) to (8) were added thereto, and after that, the mixture was mixed by a disper and dispersed by a colloid mill. After that, the component (16) was added thereto, and the mixture was degassed and poured into a container at 70°C and cooled to obtain an oil-based stick foundation.

(Example 11: Cheek rouge)

A cheek rouge was produced from the following respective components shown in Table 15.

Table 15

| (1) | kaolin | 24.0 |
| (2) | blue colored flakes of Example 1 | 0.1 |
| (3) | red iron oxide | 0.3 |
| (4) | Red 202 | 0.5 |
| (5) | ceresin | 15.0 |
| (6) | vaseline | 20.0 |
| (7) | liquid paraffin | 25.0 |
| (8) | isopropyl myristate | 15.0 |
| (9) | antioxidant | q.s.(% by mass) |

The components (1) to (4) were added to a portion of the component (7), and treated by a roller to prepare a pigment material. The remaining of the component (7) and the components (5), (6), (8), and (9) were heated and dissolved at 90°C, and mixed with the pigment material and evenly dispersed by a homo-mixer and after being dispersed, the resulting mixture was packed in a prescribed container to obtain an aimed cheek rouge.

(Example 12: Lipstick)

A lipstick was produced from the following respective components shown in Table 16.

Table 16

| (1) | hydrocarbon wax | 20 |
| (2) | candelilla wax | 3 |
| (3) | glyceryl isostearate | 40 |
| (4) | liquid paraffin | 26.8 |
| (5) | titanium dioxide | 4 |
| (6) | blue colored flakes of Example 1 | 0.2 |
| (7) | organic pigment | 5.8 |
| (8) | fragrance | 0.2 (% by mass) |

The above-mentioned components (1) to (4) were dissolved at 85°C and the components (5) to (7) were added thereto, and stirred and mixed, and after that, the component (8) was further mixed and stirred and then the mixture was packed in a prescribed container to obtain a lipstick.

(Example 13: Blue coating material composition)

Coating material compositions were produced from the following respective components shown in Tables 17 and 18.
At first the following components shown in Table 17 were dispersed for 60 minutes by using a paint shaker to obtain a dispersion vehicle.

Table 17

| | | | |
|---|---|---|---|
| (1) | alkyd resin type varnish | | 20.6 |
| (2) | melamine resin type varnish | | 10.6 |
| (3) | Swazol | | 15.6 |
| (4) | blue colored flakes of Example 3 | | 15.6 (part by mass) |

The components (5) and (6) shown in the following Table 18 were further added to and mixed with the above dispersion vehicle to produce a blue coating material composition.

Table 18

| | | |
|---|---|---|
| (5) | alkyd resin type varnish | 26.3 |
| (6) | melamine resin type varnish | 11.3 (part by mass) |

(Comparative Example 6)

A blue coating material composition was produced in the same manner as Example 13 , except that 4.7 parts by mass of the ultramarine particles of Comparative Example 1 were used in place of 15 . 6 parts by mass of the blue colored flakes of the component (4) in the coating material composition of Example 13.

The uniform dispersibility and color impression for the blue coating material compositions of Example 13 and Comparative Example 6 were judged by eye observation. The results are shown in Table 19.

Table 19

| | Uniform dispersibility | Color impression |
|---|---|---|
| Example 13 | ◎ | Transparent and clear blue with bright impression |
| Comparative Example 6 | ○ | Mat blue with no bright impression |

It was found from Table 19 that the blue coating material of the present invention is excellent in the uniform dispersibility and has excellent bright impression and very beautiful transparent blue.

(Example 14: Resin composition and resin molded product)

98% by mass of methyl methacrylate copolymer beads and 2% by mass of the blue colored flakes of Example 3 were mixed and stirred by a Henschel mixer to obtain a resin composition. A 0.5 mm thick acrylic resin molded product was produced from the composition by an extruder. The resin molded product showed clear blue with bright impression.

(Comparative Example 7)

A 0.5 mm thick acrylic resin molded product was produced in the same manner as Example 14, except that 0.6% by mass of the ultramarine particles of Comparative Example 1 was used in place of 2% by mass of the blue colored flakes used in Example 14. The resin molded product showed no bright impression and mat black color.

As being understood from Example 14 and Comparative Example 7, the resin molded product of the present invention showed transparent and clear black color with bright impression.

(Example 15: Ink composition)

The following respective components were sufficiently mixed to produce blue ink.

Table 20

| | | |
|---|---|---|
| (1) | blue colored flakes of Example 3 | 12 |
| (2) | ketone resin | 19 |
| (3) | ethanol | 59 |
| (4) | propylene glycol monomethyl ether | 10 (% by mass) |

When the ink composition was used for writing on white paper, the writing showed very beautiful blue.

**Industrial Applicability**

The blue colored flakes of the present invention can be used as a filler for cosmetics, coating materials, resin molded products, and inks.

**Claims**

1.    A blue colored flake comprising a transparent metal oxide as a mother material, and 5 to 60% by mass of ultramarine particles having a particle diameter of 30 to 3000 nm contained therein in a dispersed state.

2.    The blue colored flake according to claim 1 having an average particle diameter of 5 to 500 $\mu$m, an average thickness of 0.1 to 5 $\mu$m, and an average aspect ratio of 5 to 300.

3.    The blue colored flake according to claim 1 or claim 2, wherein the mother material enclosing the ultramarine particles comprises, as a main component, at least one member selected from silicon dioxide and aluminum oxide.

4.    A production method of the blue colored flake according to any one of claims 1 to 3 comprising the steps of:

    forming a coating film by applying a metal compound solution or metal oxide sol containing ultramarine particles dispersed therein to the surface of a base material;
    forming a flaky metal oxide by heating the coating film; and
    separating the flaky metal oxide from the base material.

5.    A cosmetic comprising the blue colored flake according to any one of claims 1 to 3.

6.    A coating material composition comprising the blue colored flake according to any one of claims 1 to 3.

7.    A resin composition comprising the blue colored flake according to any one of claims 1 to 3.

8.    An ink composition comprising the blue colored flake according to any one of claims 1 to 3.

## INTERNATIONAL SEARCH REPORT

| International application No. |
| --- |
| PCT/JP2004/013769 |

| A. CLASSIFICATION OF SUBJECT MATTER |
| --- |
| Int.Cl$^7$ C09C1/32, 3/06, C09D11/00, 201/00, C08L101/00, C08K9/02 |

According to International Patent Classification (IPC) or to both national classification and IPC

| B. FIELDS SEARCHED |
| --- |
| Minimum documentation searched (classification system followed by classification symbols) |
| Int.Cl$^7$ C09C1/32, 3/06, C09D11/00, 201/00, C08L101/00, C08K9/02 |

| Documentation searched other than minimum documentation to the extent that such documents are included in the fields searched |
| --- |
| Jitsuyo Shinan Koho 1922–1996 Toroku Jitsuyo Shinan Koho 1994–2004 |
| Kokai Jitsuyo Shinan Koho 1971–2004 Jitsuyo Shinan Toroku Koho 1996–2004 |

| Electronic data base consulted during the international search (name of data base and, where practicable, search terms used) |
| --- |
|  |

### C. DOCUMENTS CONSIDERED TO BE RELEVANT

| Category* | Citation of document, with indication, where appropriate, of the relevant passages | Relevant to claim No. |
| --- | --- | --- |
| Y | JP 6-116520 A (Nippon Sheet Glass Co., Ltd.), 26 April, 1994 (26.04.94), Claims; Par. No. [0011] (Family: none) | 1–8 |
| Y | JP 8-239310 A (Nippon Sheet Glass Co., Ltd.), 17 September, 1996 (17.09.96), Claims; Par. No. [0002] (Family: none) | 1–8 |
| Y | JP 64-9803 A (Sumitomo Chemical Co., Ltd.), 13 January, 1989 (13.01.89), Claims; page 5, upper right column, the last line to page 6, upper right column, 7th line from the bottom (Family: none) | 1–8 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 13 October, 2004 (13.10.04) | 02 November, 2004 (02.11.04) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

**INTERNATIONAL SEARCH REPORT**

| International application No. |
| --- |
| PCT/JP2004/013769 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 60-81012 A (Miyoshi Kasei Inc.),<br>09 May, 1985 (09.05.85),<br>Claims; page 1, lower right column, 3rd line<br>from the bottom to page 2, upper left column,<br>line 8<br>(Family: none) | 1-8 |

Form PCT/ISA/210 (continuation of second sheet) (January 2004)